# EUROPEAN PATENT APPLICATION

(11) **EP 3 225 241 A1**
(43) Date of publication of application: **04.10.2017**
(21) Application number: 15862594.7
(22) Date of filing: 27.11.2015
(51) Int. Cl.: A61K 31/575, A61P 3/00, A61P 5/24, A61P 19/00

(54) **AGENT FOR PREVENTING OR IMPROVING SYMPTOMS CAUSED BY IMBALANCE OF SEX HORMONES**

(30) Priority: 28.11.2014 JP 2014242165
(71) Applicant: Morinaga Milk Industry Co., Ltd., Minato-ku Tokyo 108-8384 (JP)
(72) Inventor: MISAWA, Eriko, Zama-shi Kanagawa 252-8583 (JP); YAO, Ruiqing, Zama-shi Kanagawa 252-8583 (JP); SAITO, Marie, Zama-shi Kanagawa 252-8583 (JP); TANAKA, Miyuki, Zama-shi Kanagawa 252-8583 (JP)
(74) Representative: Vossius & Partner Patentanwälte Rechtsanwälte mbB
(86) International application number: PCT/JP2015/083475
(87) International publication number: WO 2016/084956

(57) **Abstract**

Provided is a means with which it is possible to efficiently prevent or improve symptoms caused by an imbalance of sex hormones with as little pain as possible, and which can safely be taken daily. A compound selected from the group consisting of a lophenol compound and a cyclolanostane compound is used as an active ingredient of an agent for preventing or improving symptoms caused by an imbalance of sex hormones.

## Description

### TECHNICAL FIELD

The present invention relates to an agent for preventing or improving symptoms due to an imbalance of sex hormones.

### BACKGROUND ART

Hormones are chemical substances which are secreted from endocrine glands, act on target sites in a body through blood, and control activities thereof. Hormones are circulated in the whole body, but many hormones act only on specific organs or tissues. At target sites, a receptor undergoing stimulation of a hormone exists, and transmits information for causing specific action by binding of the hormone thereto. Hormones influence growth, development, reproduction and the like, and at the same time, participate in energy metabolism and homeostasis such as maintenance of the concentrations of blood components, and control the functions of organs in the whole body. Since hormones cause a very great response at an extremely small amount, when the balance thereof is disturbed, a variety of symptoms and diseases appear (Non-Patent Document 1).

A female menopausal disorder results from disturbance in the hormone balance which is caused by deterioration of the ovary function and decrease in a secretion amount of estrogen in a term of transfer from a reproductive stage to a non-productive stage. Symptoms caused by the menopausal disorder include vasomotor nervous symptoms (glow, hot flash, sweating, coldness, palpitation, short breath, edema), neuropsychiatric symptoms (headache, dizziness, insomnia, anxiety, irritation, depressive state, tinnitus, deafness, lightheadedness, stress), motor organ symptoms (lumbago, shoulder stiffness, arthralgia, myalgia, bone dysbolism), digestive organ symptoms (anorexia, nausea, diarrhea, constipation, thirst, dull feeling in the stomach, heartburn), systemic symptoms (malaise), urinary symptoms (pollakiuria, residual urine, micturition pain), reproduction organ symptoms (menstrual disorder, reduction in sexual desire), sensory symptoms (numbness, desensitization, hypersensitivity, low vision), and skin symptoms (skin drying, itching, wrinkle, dullness). Further, it has been elucidated that postmenopausal females are more likely to develop hypertension, diabetes, obesity, osteoporosis, autonomic ataxia, unidentified complaint, dementia and the like (Non-Patent Document 2).

On the other hand, increase in the menopausal disorder has been also reported in males, and many of symptoms thereof are said to be due to reduction in the testosterone concentration in blood involved in a variety of neuropsychiatric symptoms. In a male menopausal disorder, various symptoms such as vasomotor nervous symptoms (glow, hot flash, sweating, coldness, palpitation), neuropsychiatric symptoms (headache, dizziness, insomnia, anxiety, depressive state, tinnitus, dyspnea), motor organ symptoms (shoulder stiffness, arthralgia, myalgia), digestive organ symptoms (anorexia, constipation, lumbago, mouth dryness), systemic symptoms (malaise), urinary symptoms (pollakiuria, residual urine, reduction in urinary stream), reproduction organ symptoms (reduction in sexual desire), sensory symptoms (numbness, desensitization), and the like are known (Non-Patent Document 3), like females. Further, there is a report that also in males, reduction in estrogen together with reduction in testosterone occur, thus, osteoporosis is developed (Non-Patent Document 4).

Like this, in both males and females, disturbance in the sex hormone balance is generated due to influence of deterioration in the function of organs due to aging in a menopausal stage, and also in females in young generation, an imbalance of sex hormones is generated in some cases. As symptom caused by such an imbalance of sex hormones, there is premenstrual syndrome (PMS). In PMS, symptoms such as vasomotor nervous symptoms (palpitation, edema), neuropsychiatric symptoms (headache, dizziness, slight fever, anxiety, depressive state, insomnia, sleepiness, stress), motor organ symptoms (lumbago, arthralgia), digestive organ symptoms (constipation, diarrhea, abdominalgia, hyperphagia, thirst), systemic symptoms (fatigue), skin symptoms (acne) and the like appear. A severity of these symptoms is significantly different among individuals, but when symptom is severe, this hinders work and daily life in some cases.

Previously, as response to various symptoms in a menopausal stage, administration of a sexual steroidal hormone (hormone replacement therapy (HRT)) has been performed (Non-Patent Document 3).

However, in treatment in which estrogen is administered for the menopausal disorder of females, not only there is a report that an onset risk of breast cancer, venous thromboembolism, cerebral stroke, ischemic cardiac disease and the like is increased, but also troublesome administration control and patient management are required, and a variety of demerits rather than usefulness of improvement in menopausal symptoms become a problem.

Further, for the menopausal disorder of males, hormone replacement therapy with an androgen preparation comprising testosterone has been spread, but as the side effect, exacerbation of prostatic cancer, urination disorder, liver function failure, retention of fluid, exacerbation of sleep apnea syndrome and the like become a problem.

In such background, so-called phytoestrogen such as a plant containing an estrange-like acting substance which is safer and easily used, and an extract and a fraction obtained therefrom have been used. As the phytoestrogen, for example, soybean isoflavones (Patent Document 1, Patent Document 2), black cohosh (Patent Document 3), Eucommia ulmoides (Patent Document 4), root of Pueraria radix, Smilax rhizoma, Rehmanniae radix (Patent Document 5) and the like have been reported. Particularly, regarding isoflavone, equol which is a metabolite is said to be an active ingredient, and enteric bacteria are involved in conversion to equol in a body and an individual difference is great in production, hence, an equol producing composition in which a microorganism having the ability to produce equol is added to daidzeins has also been disclosed (Patent Document 6). In these phytoestrogens, it is said that the effect is relatively mild, and there is little side effect, but a possibility of an onset risk of breast cancer or uterine cancer is not denied, and upon ingestion of a supplement or the like, attention is attracted.

Further, an aromatase activator which exerts the effect of preventing, improving or treating each symptom appearing due to decrease in estrogen by activating aromatase being an enzyme of converting androgen to estrogen (Patent Document 7), and an aromatase inhibitor which exerts the effect of preventing/treating symptom due to decrease in a male hormone or breast cancer in postmenopausal females by inhibiting aromatase (Patent Document 8) are also disclosed.

On the other hand, as a material for alleviating symptom at a menopausal stage without exhibiting the hormone-like action, a cacao bean extract-containing product (Patent Document 9), Tetragonolobus plant and an extract thereof (Patent Document 10), a rhizome portion powder or an extract of Dioscoreacea plant containing diosgenin as an active ingredient (Patent Document 11), a rhizome and an extract of Vitis plant, Japanese knotweed (Patent Document 12) and the like have been disclosed.

Meanwhile, it has been found out in an arteriosclerosis model animal that among phytosterols, a compound having a cyclolanostane skeleton and a compound having a lophenol skeleton have the action of reducing the amount of lipid peroxide in blood, and have the action of suppressing the number of the plaque formation of thoracic aorta, and use as an antioxidant has been proposed (Patent Document 13). Further, a sterol obtained from a plant-derived sterol ester is known to have the pharmacological action such as the autonomic nerve activating action, the lipid-lowering action, the platelet aggregation suppressing action, the cerebral function activating action and the like, and a fat emulsion containing the sterol which can greatly exert these pharmacological actions has been disclosed (Patent Document 14). However, a relationship of a compound having a cyclolanostane skeleton or a compound having a lophenol skeleton with symptom due to disturbance in the sex hormone balance has not been reported at all.

### PRIOR ART DOCUMENTS

### NON-PATENT DOCUMENTS

Non-Patent Document 1: Riddle of Hormone Development - Short Cut for Understanding Environmental Hormones, Yasunobu EGUCHI, Ishiyaku Pub, Inc. p. 2-5, 2002
Non-Patent Document 2: Hormone and Medical Practice, Igakunosekaisha, Inc., Vol. 57, p. 949-954, 2009
Non-Patent Document 3: Base and Medical Practice of Antiaging Medicine, edited by Japanese Society of Anti-Aging Medicine, Medical Specialist Leader Qualification Committee, p. 214-224, 2006
Non-Patent Document 4: Basic Aging Study, Japan Society for Biomedical Gerontology, Vol. 34, p. 13-17, 2010

### PATENT DOCUMENTS

- Patent Document 1:: JP-A No. 2007-186483
- Patent Document 2:: JP-A No. 2005-229855
- Patent Document 3:: Japanese Patent No. 4210190
- Patent Document 4:: JP-T No. 2012-77012
- Patent Document 5:: Japanese Patent No. 4892856
- Patent Document 6:: JP-A No. 2009-232712
- Patent Document 7:: JP-A No. 2012-171933
- Patent Document 8:: JP-A No. 2014-189539
- Patent Document 9:: JP-A No. 2001-69946
- Patent Document 10:: JP-A No. 2012-31146
- Patent Document 11:: JP-A No. 2007-16013
- Patent Document 12:: Japanese Patent No. 4768105
- Patent Document 13:: WO 2010/058795
- Patent Document 14:: JP-A No. 4-91026

### DISCLOSURE OF THE INVENTION

### PROBLEMS TO BE SOLVED BY THE INVENTION

It has been confirmed that the materials disclosed in Patent Documents 9 to 12 exhibit the effect of improving symptoms which can occur at a menopausal stage, as one example of the effect on symptoms caused by an imbalance of sex hormones, but that effect is only on a part of symptoms, and the direct effect on symptoms caused by an imbalance of sex hormones has not been confirmed. Further, the effect confirmed in Patent Document 14 is only the platelet aggregation suppressing action, and there was no description regarding other pharmacological actions.

Then, an object of the present invention is to provide a functional material which can safely be ingested daily and can effectively prevent or improve symptoms caused by disturbance in the sex hormone balance, with as little pain as possible, and a agent utilizing this.

### MEANS TO SOLVE THE PROBLEMS

The first invention which solves the above-mentioned problems is an agent for preventing or improving symptoms caused by an imbalance of sex hormones, which contains, as an active ingredient, a compound selected from the group consisting of lophenol compounds and cyclolanostane compounds (hereinafter, referred to also as "agent of the present invention").

In a preferable embodiment of the present invention, the agent comprises the above-described compound at a total amount of 0.00001% by mass or more.

In a preferable embodiment of the present invention, the above-described compound is selected from the group consisting of 4-methylcholest-7-en-3-ol, 4-methylergost-7-en-3-ol, 4-methylstigmast-7-en-3-ol, 9,19-cyclolanostan-3-ol and 24-methylene-9,19-cyclolanostan-3-ol.

The agent of the present invention is effective in preventing or improving symptoms caused by an imbalance of female hormones.

In a preferable embodiment of the present invention, the agent of the present invention is an agent which prevents or improves the symptom not through the sex hormone-like action.

The symptoms caused by an imbalance of sex hormones include bone dysbolism and neuropsychiatric symptoms, and the above-described compound or the composition containing this is particularly effective in preventing or improving these symptoms.

Further, the second invention for solving the above-mentioned problem is use of a compound selected from the group consisting of lophenol compounds and cyclolanostane compounds in the manufacture of an agent for preventing or improving symptoms caused by an imbalance of sex hormones, and a preferable embodiment of the compound is as described above.

Further, the second invention includes the following embodiment.

Use of a composition containing a compound selected from the group consisting of lophenol compounds and cyclolanostane compounds at a total amount of 0.00001% by mass or more in the manufacture of an agent for preventing or improving symptoms caused by an imbalance of sex hormones.

In a preferable embodiment of the present invention, the agent is for preventing or improving symptoms caused by an imbalance of female hormones.

In a preferable embodiment of the present invention, the agent is for preventing or improving the above-described symptoms not through the sex hormone-like action.

In a preferable embodiment of the present invention, the agent is for preventing or improving bone dysbolism or neuropsychiatric symptoms.

Further, the third invention for solving the above-mentioned problem is a compound selected from the group consisting of lophenol compounds and cyclolanostane compounds, which is used for preventing or improving symptoms caused by an imbalance of sex hormones, and a preferable embodiment of the compound is as described above.

In a preferable embodiment of the present invention, the symptoms caused by an imbalance of sex hormones are symptoms caused by an imbalance of female hormones.

In a preferable embodiment of the present invention, the above-described compound is for preventing or improving the symptoms not through the sex hormone-like action.

In a preferable embodiment of the present invention, the above-described symptoms caused by an imbalance of sex hormones are bone dysbolism and neuropsychiatric symptoms.

Further, the fourth invention for solving the above-mentioned problem is a composition containing a compound selected from the group consisting of lophenol compounds and cyclolanostane compounds at a total amount of 0.00001% by mass or more, which is used for preventing or improving symptoms caused by an imbalance of sex hormones, and a preferable embodiment of the compound is as described above.

In the fourth invention, the above-described composition is preferably food or drink.

In a preferable embodiment of the present invention, the symptoms caused by an imbalance of sex hormones are symptoms caused by an imbalance of female hormones.

In a preferable embodiment of the present invention, the above-described composition is for preventing or improving the symptoms not through the sex hormone-like action.

In a preferable embodiment of the present invention, the symptoms caused by an imbalance of sex hormones are bone dysbolism and neuropsychiatric symptom.

Further, the fifth invention for solving the above-mentioned problem is a method for preventing or improving symptoms caused by an imbalance of sex hormones, comprising administering a compound selected from the group consisting of lophenol compounds and cyclolanostane compounds to a subject having symptoms caused by an imbalance of sex hormones, and a preferable embodiment of the compound is as described above.

Further, the fifth invention has the following preferable embodiment:
The composition containing a compound selected from the group consisting of lophenol compounds and cyclolanostane compounds at a total amount of 0.00001% by mass or more is administered to the above-described subject.

In a preferable embodiment of the present invention, the above-described symptoms caused by an imbalance of sex hormones are symptoms caused by an imbalance of female hormones.

In a preferable embodiment of the present invention, the prevention or the improvement is conducted not through the sex hormone-like action.

In a preferable embodiment of the present invention, the above-described symptoms caused by an imbalance of sex hormones are bone dysbolism and neuropsychiatric symptoms.

### EFFECT OF THE INVENTION

The agent of the present invention can be safely ingested, and efficiently prevents or improves symptoms caused by an imbalance of sex hormones. Particularly, the agent of the present invention is effective in preventing or improving symptoms caused by an imbalance of female hormones. Further, the agent of the present invention is effective in preventing or improving, particularly, bone dysbolism or neuropsychiatric symptoms being a symptom caused by an imbalance of sex hormones.

### DESCRIPTION OF EMBODIMENTS

Then, preferable embodiments of the present invention will be illustrated in detail. The present invention is not limited to the following preferable embodiments, and can be freely modified in the scope of the present invention. In addition, percentage in the present description is indication by mass, unless otherwise indicated. Further, the "preventing or improving agent" of the present invention (in the present description, simply referred to as "agent of the present invention" in some cases) has the same meaning as that of the "preventing or treating agent" of the present invention, and every case is included in definition of the agent of the present invention. Further, in the present description, "improvement" is the concept including "treatment".

The agent of the present invention contains, as an active ingredient, a compound selected from the group consisting of lophenol compounds (Compound 1) and cyclolanostane compounds (Compound 2). Compound 1 and Compound 2 are represented by the following general formula (1) and the general formula (2), respectively.

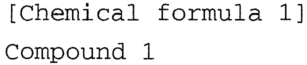

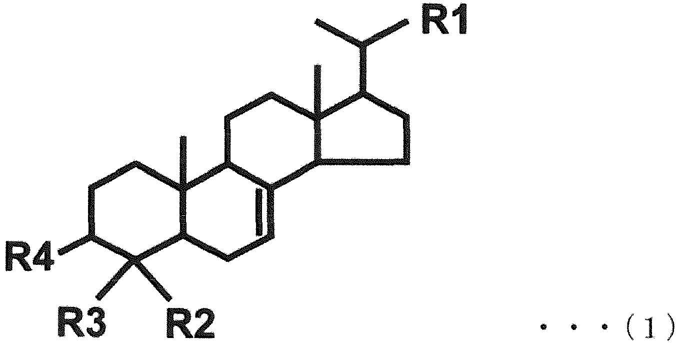

In the general formula (1), R1 is an alkyl group or an alkenyl group including one or two double bonds, which is straight or branched chain having 5 to 16 carbon atoms. The alkyl or alkenyl group may be a substituted alkyl or alkenyl group, in which one or two hydrogen atoms are substituted with a hydroxyl group and/or a carbonyl group.

R2 and R3 each are independently a hydrogen atom or an alkyl group having 1 to 3 carbon atoms. Herein, as the alkyl group having 1 to 3 carbon atoms, a methyl group, an ethyl group and the like are preferable, and a methyl group is particularly preferable. The alkyl group may be a substituted alkyl group in which at least one hydrogen atom is substituted with a hydroxyl group and/or a carbonyl group.

[Chemical formula 2] -CH₂-OH -CH₂-COOH -CH₂-CH₂-OH -CH₂-CH₂-COOH -CH(OH)-CH₃ -CH(COOH)-CH₃

R4 forms C=O with a carbon atom constituting the ring, or is -OH or -OCOCH₃.

In the general formula (1), R1 is preferably any of groups represented by the following formulae.

[Chemical formula 3] -CH₂-CH₂-CH(CH₂-CH₃)-CH(CH₃)₂ -CH₂-CH₂-CH=C(CH₃)₂ -CH₂-CH=C(CH₃)-CH(CH₃)₂ -CH₂-CH₂-C(=CH-CH₃)-CH(CH₃)₂ -CH₂-CH₂-CH(Ra)=C(CH₃)Rb

(wherein Ra and Rb are any of a hydrogen atom, a hydroxyl group and a methyl group)

-CH₂-CH₂-CH(Rc)-CH(CH₃)Rd

(wherein Rc and Rd are any of a hydrogen atom, a hydroxyl group and a methyl group)

In the general formula (1), it is preferable that one of R2 and R3 is a hydrogen atom, and the other is a methyl group, and it is preferable that R4 is a hydroxy group.

Compound 1 includes preferably 4-methylcholest-7-en-3-ol, 4-methylergost-7-en-3-ol and 4-methylstigmast-7-en-3-ol. Respective compounds have structures represented by the following formulae, respectively.

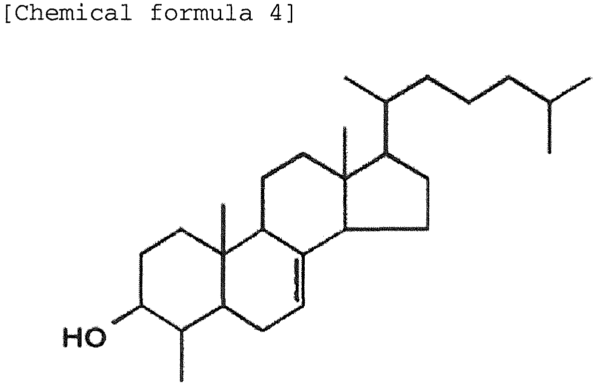

### 4-Methylcholest-7-en-3-ol

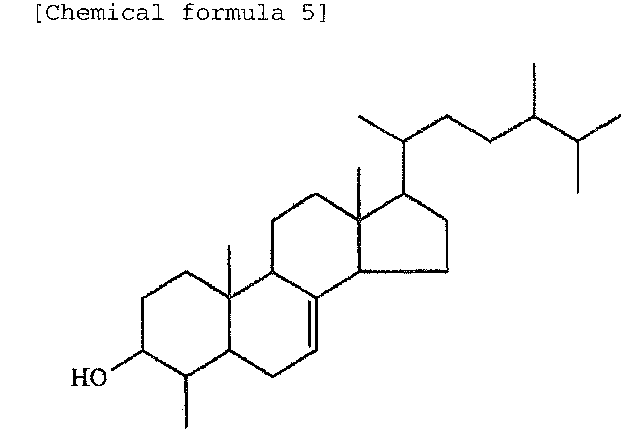

### 4-Methylergost-7-en-3-ol

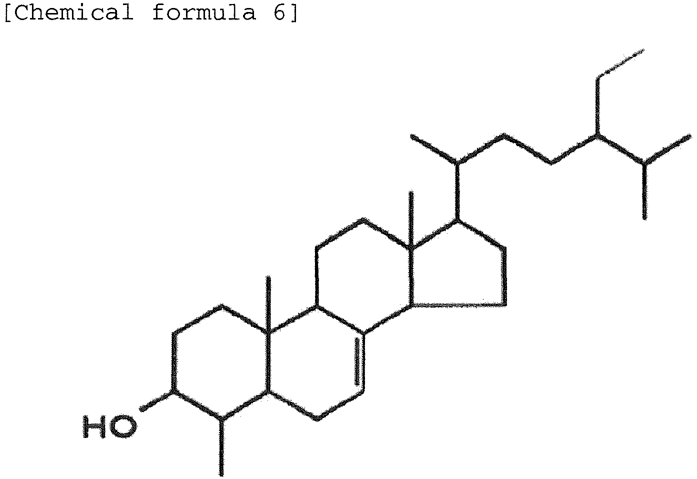

### 4-Methylstigmast-7-en-3-ol

Compound 1 can be chemically manufactured in accordance with the known manufacturing processes.

Compound 1 can be synthesized, for example, in accordance with supplement data described in Vitali Matyash et al., PLOS BIOLOGY, Volume 2, Issue 10, e280, 2004.

Further, it is known that Compound 1 is contained in plants, and Compound 1 can be manufactured in accordance with the known process for manufacturing lophenol (Biochemistry Experimental Method 24, Fat Lipid Metabolism Experimental Method, authored by Akihiro YAMADA, Gakkai Shuppan Center, p. 174, 1989).

For example, Compound 1 can be extracted from plants which are known to contain Compound 1, using a method such as a hot water extraction method, an organic solvent extraction method, a supercritical extraction method and a subcritical extraction method (see, e.g., Japanese Patent No. 3905913). Compound 1 can be extracted, for example, from plants belonging to family *Liliaceae,* family *Leguminosae,* family *Gramineae,* family *Solanaceae* and family *Musaseae.*

The molecular weight and the structure of Compound 1 manufactured as described above can be determined or confirmed by a mass spectrometry (MS), a nuclear magnetic resonance spectral (NMR) method.

Further, Compound 1 may be a pharmaceutically acceptable salt. The pharmaceutically acceptable salt includes both metal salts (inorganic salts) and organic salts, and as a list of them, that described in "Remington's Pharmaceutical Sciences, 17th edition, 1985, p. 1418" is exemplified.

Specifically, inorganic salts such as a hydrochloride, a sulfate, a phosphate, a diphosphate, and a hydrobromide, and organic salts such as a malate, a maleate, a fumarate, a tartrate, a succinate, a citrate, an acetate, a lactate, a methanesulfonate, a p-toluenesulfonate, a pamoate, a salicylate, and a stearate are included without limitation.

Meanwhile, Compound 1 may be a salt with a metal such as sodium, potassium, calcium, magnesium and aluminum, or a salt with an amino acid such as lysine. Moreover, there may also be used a solvate such as a hydrate of the compounds or pharmaceutically acceptable salts thereof.

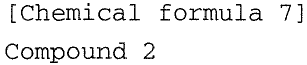

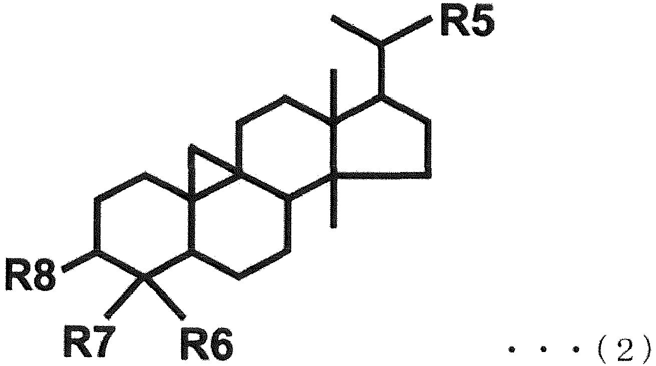

In the general formula (2), R5 is an alkyl group or an alkenyl group including one or two double bonds, which is straight or branched chain having 6 to 8 carbon atoms. The alkyl or alkenyl group may be a substituted alkyl or alkenyl group in which one or two hydrogen atoms are substituted with a hydroxyl group and/or a carbonyl group.

R6 and R7 each are independently a hydrogen atom or a methyl group. R8 forms C=O with a carbon atom constituting the ring, or is any of the following formulae.

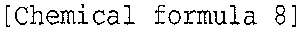

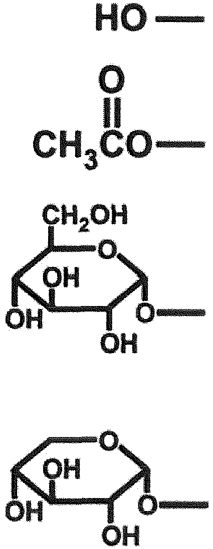

In the general formula (2), R5 is preferably any of groups represented by the following formulae.

[Chemical formula 9] -CH₂₋CH₂-CH₂-CH(CH₃)₂ -CH₂-CH₂-CHRe-C(CH₃)₂Rf

(Re is a hydrogen atom, a hydroxyl group or a methyl group, and Rf is a hydrogen atom or a hydroxyl group)

-CH₂-CH₂-CH(CH₂-CH₃)-CH(CH₃)₂

-CH₂-CH₂-CHRg-C(CH₃)=CH₂

(Rg is a hydrogen atom, a hydroxyl group or a methyl group)

-CH₂-CH₂-C(=O)-CH(CH₃)=CH₂

-CH₂-CH₂-C(=CH₂)-CH(CH₃)₂

-CH₂-CH₂-CH=C(CH₃)₂

-CH₂-CH₂=C(CH₃)-CH(CH₃)₂

-CH₂-CH₂-C(=CH-CH₃)-CH(CH₃)₂

Further, in the general formula (2), it is preferable that one of R6 and R7 is a hydrogen atom, and the other is a methyl group, and it is preferable that R8 is a hydroxy group.

Compound 2 includes preferably 9,19-cyclolanostan-3-ol and 24-methylene-9,19-cyclolanostan-3-ol. Respective compounds have structures represented by the following formulae, respectively.

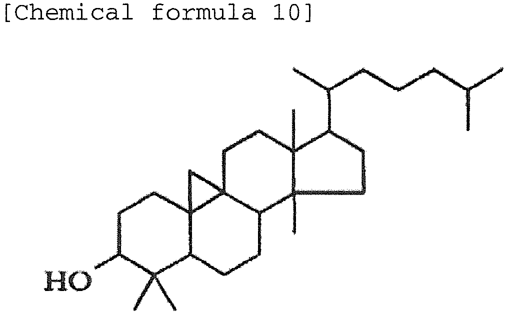

### 9,19-Cyclolanostan-3-ol

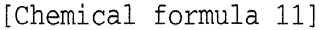

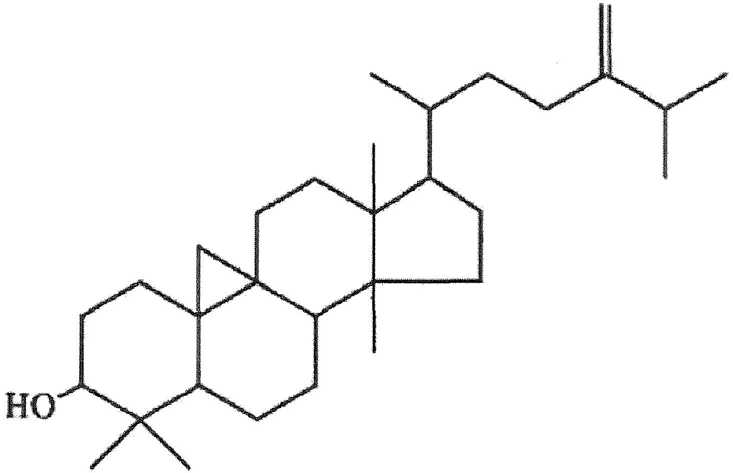

### 24-Methylene-9,19-cyclolanostan-3-ol

Compound 2 can be chemically manufactured in accordance with the known manufacturing processes. For example, 24-methylene-9,19-cyclolanostan-3-ol (trivial name: 24-methylenecycloartanol) can be manufactured by the methods disclosed in JP-A No. 57-018617 and WO 2012/023599 (method of synthesis from γ-oryzanol). Alternatively, Compound 2 can be manufactured using a hydrolysate of cycloartenol ferulate as a starting substance, by the method disclosed in JP-A No. 2003-277269.

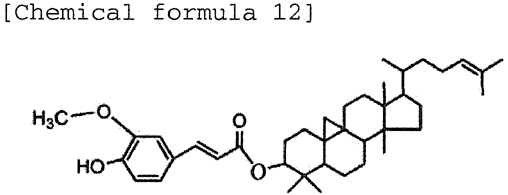

### Cycloartenol ferulate

Further, Compound 2 is also known to be contained in a plant belonging to family *Liliaceae,* family *Leguminosae,* family *Gramineae,* family *Solanaceae,* or family *Musaseae* (see [Phytochemistry, USA, 1977, vol. 16, pp. 140-141], [Handbook of phytochemical constituents of GRAS herbs and other economic plants, 1992, USA, CRC Press] or [Hager's Handbuch der Pharmazeutischen Praxis, vol. 2-6, 1969-1979, Deutschland, Springer Verlag, Berlin]). Hence, Compound 2 can be extracted from these plants using the known methods such as an organic solvent extraction method or a hot water extraction method (see, e.g., Japanese Patent No. 3924310). It is preferable that Compound 2 is extracted, for example, from plants of *Liliaceae Aloe.*

The molecular weight and the structure of the compound manufactured as described above can be determined or confirmed, for example, by mass spectrometry (MS) and nuclear magnetic resonance spectrometry (NMR).

Further, Compound 2 may be a pharmaceutically acceptable salt. Such a salt is as exemplified concerning Compound 1.

The agent of the present invention contains, as an active ingredient, a compound selected from the group consisting of Compound 1 and Compound 2. The compound may be one kind, that is, either of Compound 1 or Compound 2 alone, or may be a mixture of Compound 1 and Compound 2.

When Compound 1 or Compound 2 is used alone, either Compound 1 (mainly, 4-methylcholest-7-en-3-ol, 4-methylergost-7-en-3-ol, or 4-methylstigmast-7-en-3-ol) or Compound 2 (mainly, 9,19-cyclolanostan-3-ol or 24-methylene-9,19-cyclolanostan-3-ol) is preferable.

Among them, 4-methylcholest-7-en-3-ol is particularly preferable as Compound 1, and 9,19-cyclolanostan-3-ol is particularly preferable as Compound 2, from a view point of physical properties such as solubility which are considered when used as an active ingredient of the agent.

Further, when Compound 1 and Compound 2 are compared, Compound 1 (mainly, 4-methylcholest-7-en-3-ol, 4-methylergost-7-en-3-ol or 4-methylstigmast-7-en-3-ol) is more preferable.

Further, for each of Compound 1 or Compound 2, one kind of a compound may be used, or a plurality of compounds may be used by mixing them.

The agent of the present invention contains, as an active ingredient, preferably a compound selected from the group consisting of 4-methylcholest-7-en-3-ol, 4-methylergost-7-en-3-ol, 4-methylestigmast-7-en-3-ol, 9,19-cyclolanostan-3-ol and 24-methylene-9,19-cyclolanostan-3-ol.

The content of the compound in the agent of the present invention can be appropriately selected depending on symptoms or the like, and the total amount is preferably at least 0.00001% by mass, more preferably at least 0.0001% by mass, further preferably at least 0.0005% by mass, and particularly preferably at least 0.001% by mass. Further, the upper limit of the amount in the agent of the present invention is not particularly limited, but as the total amount, 90% by mass or less, preferably 70% by mass or less and more preferably 50% by mass or less are exemplified.

When both Compound 1 and Compound 2 are combined (mixture of Compound 1 and Compound 2), the range of the mass ratio of Compound 1 and Compound 2 includes, for example, the following:
Compound 1:Compound 2 is preferably 5:1 to 1:5, further preferably 3:1 to 1:3, and particularly preferably 2:1 to 1:2.

For example, as an example in which the compound is contained in a natural plant, it is known that Compound 1 (mainly, 4-methylcholest-7-en-3-ol, 4-methylergost-7-en-3-ol and 4-methylstigmast-7-en-3-ol) and Compound 2 (mainly, 9,19-cyclolanostan-3-ol and 24-methylene-9,19-cyclolanostan-3-ol) are contained in aloe vera.

For this reason, using aloe vera as a raw material, any of 4-methylcholest-7-en-3-ol, 4-methylergost-7-en-3-ol or 4-methylstigmast-7-en-3-ol (Compound 1) or any of 9,19-cyclolanostan-3-ol, or 24-methylene-9,19-cyclolanostan-3-ol (Compound 2) can be purified, respectively, to obtain a mixture containing Compound 1:Compound 2 at 5:1 to 1:5, preferably 3:1 to 1:3, and particularly preferably 2:1 to 1:2. Thus obtained mixture is suitable as an active ingredient of the agent of the present invention.

The agent of the present invention can be used in an aspect of a medicament.

The agent of the present invention can be orally or parenterally administered to a mammal including a human.

In the present invention, as symptoms caused by an imbalance of sex hormones, symptoms caused by a menopausal disorder, a postmenopausal disorder, a juvenile menopausal disorder, a menstrual disorder, dysmenorrhea, a sleep disorder and the like in addition to bone dysbolism, neuropsychiatric symptoms and the like can also be exemplified.

For example, symptoms caused by a menopausal disorder include vasomotor nervous symptoms (glow, hot flash, sweating, coldness, palpitation, short breath, edema), neuropsychiatric symptoms (headache, dizziness, insomnia, anxiety, irritation, depressive state, tinnitus, deafness, lightheadedness, stress, dyspnea), motor organ symptoms (lumbago, shoulder stiffness, arthralgia, myalgia, bone dysbolism), digestive organ symptoms (anorexia, nausea, diarrhea, constipation, thirst, dull feeling in the stomach, heartburn), systemic symptoms (malaise), urinary symptoms (pollakiuria, residual urine, micturition pain, reduction in urinary stream), reproduction organ symptoms (menstrual disorder, reduction in sexual desire), sensory symptoms (numbness, desensitization, hypersensitivity, low vision), skin symptoms (skin drying, itching, wrinkle, dullness) and sleep disorders (sleep apnea syndrome, etc.). Other examples include rheumatoid arthritis, goiter and the like. These symptoms are seen at a menopausal stage in males and females.

Further, symptoms caused by a postmenopausal disorder include hypertension, diabetes, obesity, osteoporosis, unidentified complaint, dementia and the like.

Further, symptoms of premenstrual syndrome include vasomotor nervous symptoms (palpitation, edema), neuropsychiatric symptoms (headache, dizziness, slight fever, anxiety, depressive state, insomnia, sleepiness, stress), motor organ symptoms (lumbago, arthralgia), digestive organ symptoms (constipation, diarrhea, abdominalgia, hyperphagia, thirst), systemic symptoms (fatigue), and skin symptoms (acne).

Inter alia, the agent of the present invention is effective for symptoms due to an imbalance of female hormones. As the female hormone, estrogen including estrone, estradiol and estriol (follicular hormone), and progesterone (corpus luteum hormone) are known. Among them, estrogen has its receptors in a variety of tissues, and acts on the reproduction function, the cardiac blood vessel, the skeleton, the skin and the like. For this reason, the agent of the present invention is particularly effective in symptoms caused by an imbalance of female hormones due to deficiency of estrogen. Such symptoms include bone dysbolism, neuropsychiatric symptoms and the like. Further, symptoms caused by a menopausal disorder, a postmenopausal disorder, a juvenile menopausal disorder, a menstrual disorder, dysmenorrhea, premenstrual syndrome and the like can also be exemplified.

In addition, symptoms caused by a female menopausal disorder include vasomotor nervous symptoms (glow, hot flash, sweating, coldness, palpitation, short breath, edema), neuropsychiatric symptoms (headache, dizziness, insomnia, anxiety, irritation, depressive state, tinnitus, deafness, lightheadedness, stress), motor organ symptoms (lumbago, shoulder stiffness, arthralgia, myalgia, bone dysbolism), digestive organ symptoms (anorexia, nausea, diarrhea, constipation, thirst, dull feeling in the stomach, heartburn), systemic symptoms (malaise), urinary symptoms (pollakiuria, residual urine, micturition pain), reproduction organ symptoms (menstrual disorder, reduction in sexual desire), sensory symptoms (numbness, desensitization, hypersensitivity, low vision) and skin symptoms (skin drying, itching, wrinkle, dullness). Other examples include hypertension, diabetes, obesity, osteoporosis, autonomic ataxia, unidentified complaint, dementia and the like which are said to be developed after menopause. Symptoms caused by a postmenopausal disorder and premenstrual syndrome are as stated above.

The agent of the present invention has the effect of preventing or improving the symptoms not through the sex hormone-like action. That is, the agent of the present invention influences symptoms caused by an imbalance of sex hormones, and exerts the action of preventing or improving the symptoms. Accordingly, the present invention can provide the excellent agent in a point that a difference in sex of a subject influences little difference in the effect of preventing or improving symptoms, and in a point of safety, for example.

The agent of the present invention is effective in preventing or improving one or a plurality of symptoms among the above-mentioned symptoms. Further, as shown in Examples described later, the agent of the present invention is particularly useful in that it has the effect of preventing or improving a plurality of symptoms among these symptoms.

The effect of the agent of the present invention can be confirmed using an ovariectomized mouse (OVX mouse) which is a model animal triggering an imbalance of sex hormones.

Since the OVX mouse triggers an imbalance of sex hormones by deficiency in sexual hormones, it develops a variety of symptoms due to this. In the OVX mouse, due to an imbalance of sex hormones, for example, bone dysbolism (reduction in the bone-specific alkaline phosphatase concentration in blood) or neuropsychiatric symptoms (increase in the corticosterone concentration in blood) are observed (EXPERIMENTAL AND THERAPEUTIC MEDICINE, v.8, p.957-967, 2014, Metab Brain Dis, (2013), v. 28, p. 77-84).

That is, when the agent is administered to the OVX mouse, if increase in the bone-specific alkaline phosphatase concentration in blood or reduction in the corticosterone concentration in blood is confirmed, it is supported that there is obtained the effect of preventing or improving symptoms due to an imbalance of sex hormones, including bone dysbolism and neuropsychiatric symptoms, by the administered agent.

The agent of the present invention exhibits the remarkable effect on prevention or improvement of motor organ symptoms such as bone dysbolism and the like and neuropsychiatric symptoms, among symptoms due to an imbalance of sex hormones. The effect of preventing or improving bone dysbolism includes the osteogenesis promoting effect. Further, the effect of preventing or improving neuropsychiatric symptom includes the effect of preventing or improving symptoms caused by ultraviolet stress.

The agent of the present invention is also effective in preventing or improving reduction in the bone density or osteoporosis due to bone dysbolism, or unidentified complaint which can be mainly caused by neuropsychiatric symptoms. Further, the agent of the present invention is also effective in preventing or improving these plural symptoms.

The form of the agent of the present invention is not particularly limited, and can be appropriately selected depending on a usage. Specifically, tablets, pills, powders, liquids, suspensions, emulsions, granules, capsules, syrups, suppositories, injections, ointments, patches, eye drops, nose drops and the like can be exemplified.

The administration term of the agent of the present invention is not particularly limited, and can be appropriately selected depending on a target disease. Further, it is preferable that the dose is determined depending on a dosage form, dose regimen, age and sex of a patient, other conditions, degree of symptom and the like.

The dose of the agent of the present invention is appropriately selected depending on a dose regimen, age and sex of a patient, degree of a disease, other conditions and the like. Usually, as the dose in terms of an amount of an active ingredient, a standard is preferably in the range of 0.0001 to 100 mg/day, more preferably 0.001 to 50 mg/day, and particularly preferably 0.01 to 10 mg/day.

The agent of the present invention may contain an additive which is generally used in a medicament. The additive includes an excipient, a binder, a disintegrating agent, a lubricant, a stabilizer, a flavoring agent, a diluent, a surfactant, a solvent for injection and the like.

The agent of the present invention can be manufactured by blending the above-described compound as an active ingredient into a medicament carrier. The agent of the present invention can be manufactured, for example, by formulating the compound together with the additive described above into a preparation.

Alternatively, the agent of the present invention can also be manufactured by formulating a compound obtained by extraction using hot water or various solvents, supercritical extraction, or subcritical extraction, using the known plant containing the compound or the like as a raw material, together with the additives into preparations.

Particularly, the agent of the present invention comprising Compound 1 and Compound 2 in the specific range of the mass ratio can be manufactured by mixing respective compounds in the above-mentioned range of the mass ratio. Alternatively, such a medicament can also be manufactured by a method of extraction using various solvents, supercritical extraction, subcritical extraction or the like, using the known plant containing a mixture containing Compound 1 and Compound 2 or the like as a raw material.

In the agent of the present invention, the above-described compound functions as an active ingredient, and has the action of preventing or improving symptoms caused by an imbalance of sex hormones. Particularly, the agent is effective for symptoms due to an imbalance of female hormones. Further, inter alia, the agent exerts the remarkable preventing or improving effect on bone dysbolism or neuropsychiatric symptoms.

The agent of the present invention can also be processed into food or drink by mixing it with raw materials which can be used in food or drink. In the present invention, "food or drink" includes feed which is ingested by animals other than a human, in addition to food or drink which is ingested by a human.

When food or drink is manufactured, the amount of the compound in them is, as the total amount, preferably at least 0.00001% by mass, more preferably at least 0.0001% by mass, further preferably at least 0.0005% by mass, and particularly preferably at least 0.001% by mass. Further, the upper limit of the amount in food or drink of the present invention is not particularly limited, but as the total amount, 90% by mass or less, preferably 70% by mass or less, and more preferably 50% by mass or less are exemplified.

Further, the amount of the compound in food or drink can also be made to be a suitable amount for the compound to be ingested in the range of preferably 0.0001 to 100 mg/day, more preferably 0.001 to 50 mg/day, and particularly preferably 0.01 to 10 mg/day, as expressed by the total amount, depending on the form thereof. Accordingly, one of preferable forms of food or drink of the present invention is used so that the compound is ingested at preferably 0.0001 to 100 mg/day, more preferably 0.001 to 50 mg/day, and particularly preferably 0.01 to 10 mg/day, as expressed by the total amount.

The food or drink is preferably a health functional food or drink. The "health functional food or drink" means a food or drink which directly or indirectly indicates the effect of preventing a disease, or the effect of reducing an onset risk of a disease, and a food or drink which was notified at Consumer Affairs Agency as indicating the functionality on a merchandise package based on scientific basis under business operator's responsibility. Examples thereof include foods or drinks which are currently sold as a food for specified health use, a food with function claims, a health supplement or the like in Japan.

The form of food or drink is not particularly limited, but drinks such as a soft drink, a carbonated drink, a nutritional drink, a fruit juice drink, a lactic acid bacteria beverage and the like (including concentrated original liquid and powder for preparation of these drinks) are particularly preferable from a view point that the compound is effectively ingested.

Further, it is preferable that the form of functional food or drink is a granule, tablet or liquid supplement, from a view point that a person who ingests it can easily grasp the ingestion amount of an active ingredient.

Further, it is also preferable that such functional food or drink is in a form with an indication of use of "for preventing or improving symptoms caused by an imbalance of sex hormones", "for restoring the sex hormone balance", "for preventing or improving bone dysbolism", or "for preventing or improving neuropsychiatric symptom" attached thereto. That is, it is preferable that the food or drink of the present invention is sold, for example, as food or drink for preventing or improving symptoms caused by an imbalance of sex hormones, containing a compound selected from the group consisting of Compound 1 and Compound 2 as an active ingredient, with use of "for preventing or improving symptoms caused by an imbalance of sex hormones" attached thereto.

The "indication" includes all indications having the function of informing consumers of the use. That is, indications which can evoke or analogize the use all correspond to the "indication", irrespective of an object of an indication, the content of an indication, a subject to be indicated, a medium and the like with which an indication is performed.

Further, the "with an indication attached thereto" refers to that an indication act of making consumers recognize the indication associated with food or drink (products) exists.

It is preferable that an indication act is such that consumers can directly recognize the use. Specifically, an act of describing the use on merchandise related to the food or drink of the present invention or a package of merchandise, and an act of describing the use on advertisement regarding merchandise, a price list or transaction documents (including those provided by electromagnetic method) are exemplified.

On the other hand, it is preferable that the content to be indicated (indication content) is an indication approved by administration or the like (for example, indication which received approval based on various institutions provided by administration, and is performed in an aspect based on such approval).

For example, indications of a health food, functional food or drink, an enteral nutritive food, a food for special dietary uses, a food with health claims, a special health food, a food with nutrient function claims, a food with function claims, a quasi-drug and the like can be exemplified. Particularly, indication approved by Consumer Affairs Agency, for example, indications approved by special health food institution, or institutions similar thereto can be exemplified. As an example of the latter, an indication as a special health food, an indication as a conditional special health food, an indication to the effect that a structure or the function of a body is influenced, a disease risk reducing indication and the like can be exemplified, more particularly, an indication as a special health food provided in Health Promotion Act, Enforcement Regulation (2003 April 30 Japanese Ordinance of the Ministry of Health, Labour and Welfare No. 86) (particularly, indication of use of health), and indications similar thereto can be mentioned as a typical example.

Phrase indicating the use is not limited to phrase of "for preventing or improving symptoms caused by an imbalance of sex hormones", "restoring the sex hormone balance.", "for preventing or improving bone dysbolism", or "for preventing or improving neuropsychiatric symptom", and it goes without saying that other phrases are included in the scope of the present invention as far as they are phrase indicating the action or the effect of alleviating or improving various symptoms caused by an imbalance of sex hormones.

Further, it is also preferable that the food or drink of the present invention includes an indication of the active ingredient, and further, an indication showing the relevancy between the use and the active ingredient, in addition to the indication of use.

The food or drink can be manufactured by blending a compound selected from Compound 1 and Compound 2 as an active ingredient. The food or drink of the present invention can be manufactured, for example, by mixing the compound into food or drink raw materials, followed by processing.

Alternatively, the food or drink can also be manufactured by processing an extract obtained by extraction using hot water or various solvents, supercritical extraction, or subcritical extraction using the known plant containing the compound or the like as a raw material, together with food or drink raw materials.

Further, when a form of the food or drink is made to be a granular, tablet-shaped or liquid supplement, it is also preferable that the compound being an active ingredient together with, for example, saccharides such as lactulose, maltitol, and lactitol, and other saccharides, for example, dextrin, starch and the like; proteins such as gelatin, soybean protein, and corn protein; amino acids such as alanine, glutamine, and isoleucine; polysaccharides such as cellulose and gum arabic; fats or oils such as soybean oil, and medium chain fatty acid triglyceride is formulated into a preparation.

### EXAMPLES

The present invention will be illustrated in more detail below by way of Examples, but the present invention is not limited to the following Examples.

### [Production Example 1]

### (Production of Lophenol Compound (Compound 1))

Mesophyll of aloe vera (transparent gel portion) (100 kg) was liquefied using a homogenizer, and 100 L of an ethyl acetate/butanol (3:1) mixed liquid was added thereto, followed by stirring. After allowing to stand overnight, the ethyl acetate/butanol mixed liquid and an aqueous layer were separated to recover the ethyl acetate/butanol mixed liquid. This ethyl acetate/butanol mixed liquid was concentrated under reduced pressure. The mass of the recovered ethyl acetate/butanol mixed liquid extract was 13.5 g.

A solution obtained by dissolving 13 g of the extract in 1 ml of a chloroform/methanol (1:1) mixed liquid was passed through a column filled with 400 g of Silica Gel 60 (manufactured by Merck & Co. , Inc.), the extract was adsorbed thereon, then, eluted by a stepwise gradient method of stepwisely increasing the methanol concentration using a chloroform/methanol mixed liquid (each mixing ratio of chloroform: methanol=100: 1, 25:1, 10:1, 5:1 and 1:1), and the eluted liquid was fractionated for every mixed ratio of the mixed liquid. It was confirmed by normal phase and reverse phase thin layer chromatography (manufactured by Merck & Co., Inc., Silica Gel 60F254 and RP-18F2543) that the lophenol compound of the present invention exists in a fraction which had been eluted at chloroform: methanol=25:1, among these fractions.

The solvent of this fraction was removed, then, the residue was dissolved in 1 ml of a chloroform/methanol (1:1) mixed liquid, passed through a column filled with 100 g of Silica Gel 60, adsorbed onto a column, then, eluted with 1100 ml of a hexane/ethyl acetate (4:1) mixed liquid. Eluted fractions were collected by 300 ml (Fraction A), 300 ml (Fraction B), and 500 ml (Fraction C) in this order.

It was confirmed by normal phase and reverse phase thin layer chromatography that the lophenol compound being Compound 1 of the present invention was concentrated in Fraction A, and further separated with a chloroform/hexane (85:15) mixed liquid using HPLC equipped with COSMOSIL C18 (manufactured by Nacalai Tesque, Inc.) to obtain 4-methylcholest-7-en-3-ol, 4-methylergost-7-en-3-ol, and 4-methylstigmast-7-en-3-ol at 1.3 mg, 1.2 mg, and 1 mg, respectively. The structure of each compound was confirmed by mass spectrometry(MS) and NMR.

### [Production Example 2]

### (Production of Cyclolanostane Compound (Compound 2))

To 8.0 g of γ-oryzanol (manufactured by Oryza Oil & Fat Chemical Co., Ltd.) were added 250 ml of distilled water, 50 g of sodium hydroxide, 150 ml of isopropanol, 150 ml of ethanol, and 150 ml of methanol, and heating refluxing was performed for 2 hours using a mantle heater. After the reaction, the reaction liquid was added to 1300 ml of water, and the produced white precipitate was suction-filtered to obtain a solid. In order to wash the remaining alkali, the resulting residue was suspended in 1000 ml of water, and suction filtration was performed again. This operation was repeated two times, and the final residue was lyophilized under reduced pressure to obtain 5.91 g of an oryzanol hydrolysate. The hydrolysate was purified by HPLC to obtain 2435 mg of cycloartenol, and 1543 mg of 24-methylene-9,19-cyclolanostan-3-ol (Compound 2).

Then, using the resulting cycloartenol, 9,19-cyclolanostan-3-ol (Compound 2) was synthesized.

302 mg of cycloartenol, 150 ml of isopropanol, and 1.0 g of a powdery 5% palladium-carrying carbon catalyst were charged, these were sealed in an autoclave, the interior thereof was replaced with a nitrogen gas, and a hydrogen gas was introduced while applying a pressure of 3 kg/cm². This was heated while stirring, and at the time point at which a temperature became 50°C, a pressure of hydrogen was adjusted at 5 kg/cm², and a reaction was performed for 6 hours while retaining a pressure by supplementing absorbed hydrogen. The reaction liquid was filtered to remove the catalyst, concentrated and purified by silica gel column chromatography (developing solvent: chloroform 100%) to obtain 275 mg of 9,19-cyclolanostan-3-ol.

### [Production Example 3]

### (Preparation of sample containing mixture of lophenol compound and cyclolanostane compound added thereto)

Using 4-methylcholest-7-en-3-ol, 4-methylergost-7-en-3-ol and 4-methylstigmast-7-en-3-ol which had been obtained by Production Example 1, and 9,19-cyclolanostan-3-ol and 24-methylene-9,19-cyclolanostan-3-ol which had been obtained by Production Example 2, a mixture in which the mass ratio of the lophenol compound and the cyclolanostane compound was lophenol compound (Compound 1):cyclolanostane compound (Compound 2) = 1:1 was obtained.

Using carboxymethylcellulose (CMC: manufactured by DKS Co., Ltd.), a mixture of the lophenol compound and the cyclolanostane compound was dispersed to prepare a 10000-fold diluted powder and a 100000-fold diluted powder. These powders were added to the AIN93G feed at 2%, to prepare a test sample 1 and a test sample 2.

That is, a test sample 1 containing Compound 1 (lophenol compound) and Compound 2 (cyclolanostane compound) at a total amount of 0.00002% by mass, and a test sample 2 containing these compounds at a total amount of 0.000002% by mass were produced.

### [Example 1]

In the present Example, using a hairless mouse which had been ovariectomy (OVX) -treated, which is a model triggering an imbalance of sex hormones, the osteogenesis promoting action of a composition containing Compound 1 and Compound 2 was studied.

### (1) Preparation of Sample

The test sample 1 and the test sample 2 obtained in Production Example 3, as well as a sample containing Compound 1 (lophenol compound) alone at 0.00002% by mass (test sample 3) and a sample containing Compound 2 (cyclolanostane compound) alone at 0.00002% by mass (test sample 4) were used. Further, a sample containing Compound 1 (lophenol compound) alone at 0.00001% by mass (test sample 5) and a sample containing Compound 2 (cyclolanostane compound) alone at 0.00001% by mass (test sample 6) were also prepared. In addition, as a control sample, an animal feed AIN93G was used.

### (2) Test Method

Female Hos: HR-1 mice which had been subjected to ovariectomy operation (OVX) at 8 week-old (OVX mice), and female Hos: HR-1 mice which had been subjected to sham operation (Sham) at 8 week-old (Sham mice) were purchased at 9 week-old from Japan SLC, Inc. Both of OVX mice and Sham mice were pre-reared with an animal feed AIN93G for 2 weeks, a control sample (AIN93G) was given to Sham mice (6 animals) (Sham group (negative control)), OVX mice were divided into a total of 5 groups, one group consisting of 6 animals, a control sample was given to one group (OVX group (positive control)), a test sample 1, a test sample 2, a test sample 3, or a test sample 4 was given to remaining four groups, respectively (OVX + test sample group), and animals were reared for 8 weeks.

At completion of a rearing term, blood was collected from each mouse to obtain serum, a bone-specific alkaline phosphatase (BALP) value in blood was measured using ELISA Kit of Company CUSABIO, and the action of the cyclolanostane compound or the lophenol compound on osteogenesis was assessed.

### (3) Test Result

The bone-specific alkaline phosphatase concentration in blood at 8 weeks from start of administration is shown in Table 1. In Table, a value is shown as average of one group of 6 animals ± standard deviation, and a p value in Table shows a significance probability by Student t-test.

As shown in Table 1, as compared with the Sham group being a negative control, in the OVX group being a positive control, the bone-specific alkaline phosphatase concentration in blood showed a low value. From this, it was seen that by ovariectomy, hormones are deficient, the hormone balance is disturbed, and reduction in the bone-specific alkaline phosphatase concentration in blood occurs.

On the other hand, in the OVX group to which the test sample 1 had been given, the bone-specific alkaline phosphatase concentration in blood showed a significantly high value as compared with the OVX group being a positive control. Thereby, it was seen that by ingesting a composition containing the cyclolanostane compound and the lophenol compound in the state where the bone-specific alkaline phosphatase concentration in blood is reduced due to ovariectomy, the bone-specific alkaline phosphatase concentration in blood is increased. In addition, also in the OVX group to which the test sample 2 had been given, the same effect as that of the OVX group to which the test sample 1 had been given was confirmed, although not included in Table 1.

Further, also concerning the bone-specific alkaline phosphatase concentration in blood in the OVX group to which the test sample 3 or the test sample 4 had been given, the same degree of the effect as that of test sample 1 or the test sample 2 was confirmed, in every case (data not shown).

Further, also concerning the bone-specific alkaline phosphatase concentration in blood in the OVX group to which the test sample 5 or the test sample 6 had been given, the same degree of the effect as that of test sample 1 or the test sample 2 was confirmed, in every case (data not shown).
[Table 1]

**Table 1 Bone-specific alkaline phosphatase concentration in blood**

| Test group | BALP (ng/ml) | p value | |
|---|---|---|---|
| Sham | 2967.24 ± 262.64 | | |
| OVX | 2591.07 ± 352.17 | 0.07362882 | vs Sham |
| OVX + test sample 1 | 3308.01 ± 529.43 | 0.02274307 | vs OVX |

### [Example 2]

In the present Example, stress load of ultraviolet irradiation was given to a hairless mouse which had been ovariectomy (OVX)-treated, and the anti-neuropsychiatric symptom action of a composition containing Compound 1 and Compound 2 was studied.

### (1) Preparation of Sample

The control sample used in Example 1, the test sample 1 and the test sample 2 produced in Production Example 3, as well as the test sample 3 and the test sample 4 were used.

### (2) Test Method

After OVX mice and Sham mice were pre-reared with an animal feed AIN93G for 2 weeks from 9 week-old, the control sample (AIN93G) was given to Sham mice (6 animals) (Sham group (negative control)), OVX mice were divided into a total of five groups, one group consisting of 6 animals, the control sample was given to one group (OVX group (positive control)), the test sample 1, the test sample 2, the test sample 3, or the test sample 4 was added to the remaining four groups, respectively (OVX + test sample group), and animals were reared for 8 weeks. Further two groups were made, one group consisting of 6 OVX mice, the test sample 5 or the test sample 6 was given, respectively (OVX + test sample group), and animals were reared for 8 weeks.

For each group, after completion of pre-rearing, stress loading (ultraviolet irradiation) was performed three times per week, for 6 weeks from 3 weeks from start of rearing with the control sample or the test sample. Ultraviolet irradiation was initiated at a dose of 50 mJ/cm², a dose was stepwisely increased to 125 mJ/cm², and a final total irradiation dose was 1.7 J/cm².

Twenty-four hours after final irradiation, blood was collected to obtain serum, and a corticosterone value in blood was measured using ELISA Kit of Company Enzo.

### (3) Test Result

The corticosterone concentration in blood 24 hours after final irradiation is shown in Table 2. In Table, a value is shown as average of one group of 6 animals ± standard deviation and a p value in Table shows a significance probability by Student t-test.

As shown in Table 2, as compared with the Sham group being a negative control, in the OVX group being a positive control, the corticosterone concentration in blood showed a significantly high value. From this, it was seen that by ovariectomy, hormones are deficient, the hormone balance is disturbed, and increase in the corticosterone concentration in blood occurs.

On the other hand, in the OVX group to which the test sample 1 or the test sample 2 had been given, the corticosterone concentration in blood showed a significantly low value as compared with the OVX group being a positive control. From this, it was seen that in the state where the corticosterone concentration in blood is increased due to ovariectomy, the corticosterone concentration in blood can be reduced by ingesting a composition containing the cyclolanostane compound and the lophenol compound.

Further, also concerning the corticosterone concentration in blood in the OVX group to which the test sample 3 or the test sample 4 had been given, in every case, the same degree of the reducing effect as that of the test sample 1 or the test sample 2 was confirmed (data not shown).

Further, also concerning the corticosterone concentration in blood in the OVX group to which the test sample 5 or the test sample 6 had been given, in every case, the same degree of the reducing effect as that of the test sample 1 or the test sample 2 was confirmed (data not shown).
[Table 2]

**Table 2 Corticosterone concentration in blood**

| Test group | Corticosterone (ng/ml) | p value | |
|---|---|---|---|
| Sham stress load | 131.01 ± 39.38 | | |
| OVX stress load | 180.63 ± 43.15 | 0.030858 | vs Sham |
| OVX + test sample 1 stress load | 148.90 ± 43.00 | 0.199837 | vs OVX |
| OVX + test sample 2 stress load | 144.77 ± 47.24 | 0.174436 | vs OVX |

### [Example 3]

In the present Example, the presence or absence of the estrogen-like action of a composition containing a compound selected from the cyclolanostane compound and the lophenol compound as an active ingredient, in a hairless mouse which had been ovariectomy (OVX)-treated was studied.

### (1) Preparation of Sample

The control sample, the test sample 1, the test sample 2, the test sample 3 and the test sample 4 used in Example 1 or 2 were used.

### (2) Test Method

Mice which finished rearing under the same conditions as those of each test group of Example 2 were dissected, the uterus was isolated at dissection, and a weight thereof was measured.

### (3) Test Result

A weight of the uterine at dissection is shown in Table 3.

As compared with the Sham group being a negative control, in the OVX group being a positive control, a significant reduction in a weight of the uterine was recognized, but variation in a weight of the uterine (increasing trend) was not confirmed by administration of the test sample 1, the test sample 2, the test sample 3, or the test sample 4, in every case. Further, this phenomenon did not undergo influence by stress load. From the above-mentioned result, it was suggested that the lophenol compound and/or the cyclolanostane compound have no estrogen-like action.
[Table 3]

**Table 3 Uterus weight**

| Test group | Uterus weight (mg) | mg/g body weight |
|---|---|---|
| Sham | 195.9 ± 55.7 | 7.18 |
| OVX | 35.2 ± 9.8 | 1.09 |
| OVX + test sample | 37.5 ± 12.4 | 1.21 |
| Sham stress load | 174.2 ± 55.6 | 6.58 |
| OVX stress load | 32.3 ± 11.4 | 0.96 |
| OVX + test sample 1 stress load | 35.8 ± 5.7 | 1.13 |
| OVX + test sample 2 stress load | 35.4 ± 9.0 | 1.12 |

From the above-mentioned Examples, it was seen that the agent of the present invention is effective in improving symptoms caused by disturbance in the sex hormone balance. Particularly, it was seen that the agent of the present invention is effective in improving bone dysbolism and neuropsychiatric symptom caused by disturbance in the sex hormone balance. Further, it was suggested that the agent of the present invention has no sex hormone-like action. That is, it was seen that the agent of the present invention acts on bone dysbolism and neuropsychiatric symptom, and exerts the preventing or improving action on the symptoms. For this reason, it was seen that the agent of the present invention is expected to have the preventing or improving not only symptoms due to deficiency in female hormones such as estrogen, but also the similar symptoms due to other cause. Further, since the agent of the present invention has no sex hormone-like action, it was also seen that it has high safety.

### [Example 4]

A medicament having the effect of preventing and improving symptoms due to an imbalance of the sex hormones, consisting of the following composition, was produced by the following method.

Two percent (2%) by mass of a composition prepared by adding carboxymethylcellulose (CMC: manufactured by DKS Co., Ltd.) to a mixture containing the lophenol compound produced in Production Example 1 and the cyclolanostane compound produced in Production Example 2 at a ratio of lophenol compound: cyclolanostane compound = 1:1 and dispersing the materials, the composition containing 0.001% by mass of the mixture, 2% by mass of a medium chain fatty acid (MCT: manufactured by RIKEN VITAMIN CO., LTD.), 4% by mass of a glycerin fatty acid ester (manufactured by RIKEN VITAMIN CO., LTD.), 0.5% by mass of saponin (manufactured by MARUZEN PHARMACEUTICALS CO., LTD.), 0.2% by mass of ethanol (manufactured by Japan Alcohol Corporation), 1.3% by mass of maltitol (manufactured by HAYASHIBARA CO. , LTD.), 78% by mass of glycerin (manufactured by NOF CORPORATION) and, further, water were added and the materials were mixed so that the total amount was 100% by mass, to produce a syrup-like preparation containing the mixture of the lophenol compound (Compound 1) and the cyclolanostane compound (Compound 2) at a final concentration of 0.00002% by mass.

The medicament of the present Example is effective for improving symptoms caused by disturbance in the sex hormone balance.

### INDUSTRIAL APPLICABILITY

The present invention can be utilized for preventing or improving symptoms caused by an imbalance of sex hormones.

## Claims

1. An agent for preventing or improving symptoms caused by an imbalance of sex hormones, comprising a compound selected from the group consisting of a lophenol compound and a cyclolanostane compound, as an active ingredient.

2. The preventing or improving agent according to claim 1, comprising the compound at a total amount of 0.00001% by mass or more.

3. The preventing or improving agent according to claim 1 or 2, wherein the sex hormones are female hormones.

4. The preventing or improving agent according to any one of claims 1 to 3 , wherein the agent is for preventing or improving the symptoms not through the sex hormone-like action.

5. The preventing or improving agent according to any one of claims 1 to 4, wherein the symptom caused by an imbalance of sex hormones is bone dysbolism.

6. The preventing or improving agent according to any one of claims 1 to 4, wherein the symptom caused by an imbalance of sex hormones is a neuropsychiatric symptom.

7. Use of a compound selected from the group consisting of a lophenol compound and a cyclolanostane compound in the manufacture of an agent for preventing or improving symptoms caused by an imbalance of sex hormones.

8. A compound selected from the group consisting of a lophenol compound and a cyclolanostane compound, for use in preventing or improving symptoms caused by an imbalance of sex hormones.

9. A composition comprising a compound selected from the group consisting of a lophenol compound and a cyclolanostane compound at a total amount of 0.00001% by mass or more, for use in preventing or improving symptoms caused by an imbalance of sex hormones.

10. The composition according to claim 9, wherein the composition is food or drink.

11. A method for preventing or improving symptoms caused by an imbalance of sexual hormones, comprising administering a compound selected from the group consisting of a lophenol compound and a cyclolanostane compound to a subject in need of prevention or improvement of symptoms caused by an imbalance of sex hormones.
